# EUROPEAN PATENT SPECIFICATION

(11) **EP 4 534 025 B1**
(45) Date of publication and mention of the grant of the patent: **09.12.2026**
(21) Application number: 24204778.5
(22) Date of filing: 04.10.2024
(51) Int. Cl.: A61B 17/17, A61B 17/70, A61B 17/80

(54) **TEMPLATE FOR FRACTURE REDUCTION AND POSITIONING OF PLATES IN OSTEOSYNTHESIS**
SCHABLONE ZUR BRUCHREDUKTION UND POSITIONIERUNG VON PLATTEN BEI DER OSTEOSYNTHESE
GABARIT POUR LA RÉDUCTION DE FRACTURE ET LE POSITIONNEMENT DE PLAQUES EN OSTÉOSYNTHÈSE

(30) Priority: 04.10.2023 US 202363542448 P
(43) Date of publication of application: 09.04.2025
(73) Proprietor: Stryker European Operations Limited, Carrigtwohill, Co. Cork T45 HX08 (IE)
(72) Inventor: PEREY, Bertrand, British Columbia, V3L 0E4 (CA); MCKEE, Michael, Paradise Valley, Arizona 85253 (US); SCHEMITSCH, Emil, Thornhill, Ontario L3T 1G5 (CA); DIGESER, Denis, 79111 Freiburg (DE); BLÜCHEL, Tobias, 2545 Selzach (CH); LEYENDECKER, Matthias, 79115 Weingarten (DE); MUJAWAR, Arifmohamad Hamaju, 416301 Sangli Maharashtra (IN)
(74) Representative: Röthinger, Rainer

(56) References cited:
- WO-A1-2018/081545
- WO-A1-2018/136427
- US-A1- 2014 163 570
- US-A1- 2014 163 621
- US-A1- 2017 049 493
- US-B2- 7 998 180

## Description

### REFERENCE TO RELATED APPLICATIONS

The present application claims the benefit of the filing date of U.S. Provisional Patent Application No. 63/542,448 filed on October 4, 2023.

### FIELD OF THE INVENTION

The present invention relates generally to bone fracture reduction, and in particular to devices and systems for proper alignment of a bone plate to be fixed to a fractured bone.

### BACKGROUND OF THE INVENTION

Plates for osteosynthesis, e.g., distal radius plates, are often fixed to bone across a bone fracture and substantially parallel to a longitudinal bone axis using screws or other fixation elements. Such plates are firmly fixed to a plurality of bone parts or fragments to prevent their movement relative to each other. Before fixing the plates, the bone parts or fragments to be joined or rejoined to each other should be properly positioned relative to each other to reduce the bone fracture and to promote proper alignment of the bones upon healing of the fracture.

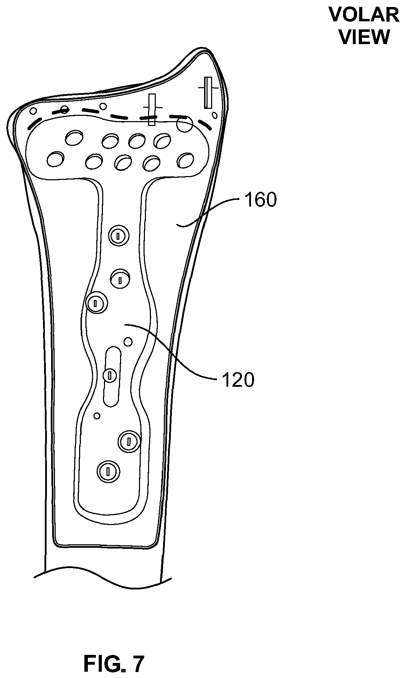

Inadequate fracture reduction and improperly positioned bone plates, especially plates placed too distally along the epiphysis of a long bone, can impair soft tissue, e.g., cause a flexor pollicis longus (FPL) tendon rupture, present a misaligned articular surface along an arthrodesis joint which could lead to later arthritic conditions, and lead to improper bone formation. Currently, verification of bone fracture reductions and bone plate placements onto bone are evaluated using fluoroscopy. Bone plates such as distal radius plates are not configured to follow the distal outline of the epiphyses of long bones but rather the distal contour of a bone, e.g., the theoretical line marking the most volar aspect of the volar margin of a radius, i.e., the so-called "watershed line" (see FIG. 7 with respect to the watershed line). As such, proper bone plate placement heavily relies on the ability and experience level of the surgeon, and thus may require multiple plate placement iterations before proper plate placement is verified. Such additional iterations require more staff and equipment resources and increase the potential for infection of patients exposed during open bone reductions. US 2014/0163621 A1 describes a bone fixation system, including methods, apparatus, and kits, and comprising a bone plate and an instrument that attaches to the bone plate and provides a radiopaque region to facilitate positioning the bone plate on bone visualized by radiographic imaging. US 2017/0049493 A1 describes a shoe for helping to position and fix a surgical implant which is intended to be attached to osseous bodies of a patient, for example those of the forearm, said shoe having a main body. WO 2018/081545 A1 describes a flexible plastic, resin or polymer material forming a trial plate for use in surgery of bones including spine and extremities. The trial plate having a radio opaque region and guides for positioning. US 7,998,180 B2 describes an apparatus, systems and methods for making, using, implanting, and visualizing portions of a medical implant. One such implant includes a bone plate at least partially of a radiolucent material having at least one aperture, and a fastener at least partially of a radiolucent material to extend through the aperture. The fastener has a distal end and a proximal end, with at least a portion of the proximal end is of a radiopaque material. A fastener lock is included to engage the fastener proximal end to maintain the fastener position relative to the bone plate. At least a portion of the fastener lock is of a radiopaque material. US 2014/0163570 A1 describes a surgical device. The device has an assembly used to position a patient-specific surgical instrument with a bone of a patient, the assembly having radio-opaque and radiolucent portions that provide an indication of the instrument, and corresponding implant's alignment with respect to the bone, as well as an indication that the angle of the projection (or orientation of the view) of the alignment guide to assess the placement of the alignment guide relative to the underlying bony anatomy. The radio-opaque portion may include a plurality of Kirschner wires, fiducial markers, and may include a void allowing for alignment down a first length of the radio-opaque portion.

Accordingly, there exists a need for a way in which to more easily and quickly align bone plates properly relative to bones to be repaired.

### SUMMARY OF THE INVENTION

The present invention provides a bone plate alignment template as defined in claim 1 and an orthopedic repair system as defined claim 8, Preferred embodiments are defined by dependent claims.

In some arrangements according to the foregoing aspect, the main body may define a central portion of the alignment template.

In some arrangements according to the foregoing aspect, the bone plate alignment template may further include distal and proximal ends and a central opening within the main body. In such arrangements, the central opening may extend across a majority of a length and a majority of a width of the distal end.

In some arrangements according to the foregoing aspect, the distal end may define a generally fan-shaped portion and may include an extension portion that may extend distally from one side of the fan-shaped portion. In some such arrangements, the bone plate alignment template may further include a distal hole that may extend through the central portion. In such arrangements, the distal hole may be between the extension portion and the central opening. In some such arrangements, the distal hole may be an oblong hole. In some such arrangements, the distal hole may be configured for receiving and tilting a K-wire.

In some arrangements according to the foregoing aspect, the bone plate alignment template may further include a proximal hole proximal the central opening.

In some arrangements according to the foregoing aspect, the template may define a longitudinal axis. In such arrangements, the bone plate alignment template may further include radiopaque indicia on opposing sides of an edge of the main body and an alignment hole through the main body. In such arrangements, the radiopaque indicia and a center of the alignment hole may lie along an axis transverse to the longitudinal axis. In some arrangements, the alignment hole may be configured for receiving a K-wire. In some such arrangements, the alignment hole may have a diameter that may be the same or approximately the same as the diameter of a corresponding K-wire. In some arrangements, the template may include an alignment feature extending from the main body into the central opening.

In accordance with another aspect, an orthopedic repair system includes a bone plate and an alignment template according to the foregoing aspect described herein. The alignment template includes a bone-facing lower surface, an upper surface opposite the lower surface, and a cavity extending inwardly from the lower surface towards the upper surface. The cavity is configured to receive a portion of the bone plate and includes a wall corresponding to a side surface of the bone plate such that the wall closely surrounds the bone plate and the bone plate moves either one of or both longitudinally and laterally with the alignment template when the bone plate is received in the cavity. In some such arrangements, the wall may tightly surround the bone plate when the bone plate is received in the cavity of the alignment template.

In some arrangements according to the foregoing aspect, the alignment template may include a central opening and the bone plate may include a set of bone plate holes. In some such arrangements, the orthopedic repair system may further include an aiming block that may be configured for receipt in the central opening. In some such arrangements, the aiming block may include a plurality of block holes that may be configured for alignment with the bone plate holes when the bone plate is received in the cavity of the alignment template and the aiming block is received in the central opening. In some such arrangements, the aiming block may have a profile that may corresponding to a profile of the central opening. In some arrangements, the orthopedic repair system may further include a first fastener that may be configured to attach the aiming block to the bone plate. In some such arrangements, the first fastener may be a set screw.

In some arrangements according to the foregoing aspect, the bone plate may include an oblong hole and the alignment template may include a corresponding oblong guide hole proximal to the central opening and configured for alignment with the oblong hole of the bone plate.

In some arrangements according to the foregoing aspect, the bone plate may include a central window defined in part by a partial hole. In some such arrangements, the alignment template may include a guide hole that may be configured for alignment with the partial hole of the central window when the bone plate is received in the cavity of the alignment template. In some such arrangements, the partial hole may include threading that may be configured for a threaded connection with a fixation screw having a shank and a threaded head extending from the shank.

In some arrangements according to the foregoing aspect, the alignment template may include a guide hole. In some such arrangements, the orthopedic repair system may further include a drill guide. The drill guide may include a shaft and a flange extending around the shaft. In some arrangements, the drill guide may be configured for being simultaneously received through the guide hole of the alignment template and attached to the bone plate such that the alignment template may be held between the flange of the drill guide and the bone plate. In some such arrangements, the guide hole of the alignment template may include a step. In such arrangements, the step may lie between the flange and the bone plate when the drill guide is received through the guide hole and attached to the bone plate. In some arrangements, the flange may extend from a location along the shaft of the drill guide such that the flange may be spaced from the step when the drill guide is received through the guide hole and attached to the bone plate. In some arrangements, the alignment template may include an alignment feature extending into the central opening. In such arrangements, the alignment feature may be configured to be received by an alignment opening of the aiming block.

In accordance with an example useful for understanding the invention and not being a part thereof, a fractured bone may be reduced by a process. In such a process, a bone plate may be placed onto or over an alignment template. In this process, a distal portion of the alignment template may be aligned to an epiphysis of the fractured bone while the alignment template is placed onto or over the bone plate. In this process, at least one locking screw may be inserted through the alignment template and into the epiphysis and the bone plate to attach the bone plate to the epiphysis. In this process, at least one locking screw may be inserted into the bone plate and a diaphysis of the bone plate to attach the bone plate to the diaphysis. In this process, the alignment template may be removed from the bone plate.

In some arrangements according to the foregoing example, a K-wire may be inserted into the epiphysis of the bone.

In some arrangements according to the foregoing example, the K-wire may be tilted to move the epiphysis bone towards the diaphysis bone.

In some arrangements according to the foregoing example, the alignment template may be moved to cause the epiphysis bone to move towards the diaphysis bone.

In some arrangements according to the foregoing example, an aiming block may be attached to the bone plate. In some such arrangements, the aiming block may be further attached to the alignment template.

In some arrangements according to the foregoing example, the aiming block may be inserted into a central opening of the alignment template and onto the bone plate such that the aiming block may fill the central opening while the aiming block is supported by the bone plate.

In accordance with another example useful for understanding the invention and not being a part thereof, a distal radius bone plate may include a distal portion, a proximal portion, and a central window. The distal portion may have at least one first fastener hole. The proximal portion may have at least one second fastener hole. The central window may be between the distal and the proximal holes. The central window may include a central opening, a proximal opening proximal to the central opening in which the proximal opening may define a partial regular hole, and a distal opening distal to the central opening in which the distal opening may define a partial regular hole.

In some arrangements according to the foregoing example, either one of or both the proximal opening and the distal opening may be partial circles.

In some arrangements according to the foregoing example, the proximal portion may include a longitudinally oriented slot and a transverse slot oriented transverse to the longitudinally oriented slot.

In some arrangements according to the foregoing example, either one of or both the proximal and the distal openings may extend over an angle greater than 180 degrees.

In some arrangements according to the foregoing example, the proximal and the distal openings may be threaded.

In some arrangements according to the foregoing example, the central opening may be wider than the proximal and the distal openings.

In some arrangements according to the foregoing example, the proximal portion of the plate may be symmetrical and define a longitudinal axis and the distal opening may define a central axis. In such arrangements, the central axis may be offset from the longitudinal axis.

### BRIEF DESCRIPTION OF THE DRAWINGS

A more complete appreciation of the subject matter of the present invention and various advantages thereof may be realized by reference to the following detailed description, in which reference is made to the following accompanying drawings, in which:
FIG. 1A includes a perspective view of bone plate alignment system on a bone along with radiographic views of an alignment template and bone plate of the alignment system on the bone in accordance with aspects;
FIG. 1B is a perspective view of another bone plate alignment system in accordance with aspects;
FIGS. 2A and 2B are plan views of the bone plate of the bone plate alignment system of FIG. 1;
FIG. 3A is a plan view of the alignment template of the bone plate alignment system of FIG. 1;
FIGS. 3B and 3C are plan views of another alignment template and aiming block in accordance with aspects;
FIG. 4 is a cross-sectional perspective view of the bone plate, the alignment template, an aiming block, and a drill guide of the bone plate alignment system of FIG. 1;
FIG. 5 is a cross-sectional elevation view of the bone plate, the aiming block, and the drill guide of the bone plate alignment system of FIG. 1;
FIG. 6 is a schematic plan view illustrating a relationship of radiopaque frame on the alignment template of the bone plate alignment system of FIG. 1;
FIG. 7 is a plan view of a combination of a bone plate and an alignment template in accordance with aspects;
FIGS. 8A-8C are plan, cross-sectional elevation, and exploded views of a bone plate alignment system in accordance with another aspect, including the bone plate and alignment template of FIG. 7; and
FIGS. 9A-9F are illustrations showing various steps of both direct and indirect bone fracture reduction processes in accordance with examples useful for understanding the invention and not being a part thereof.

### DETAILED DESCRIPTION

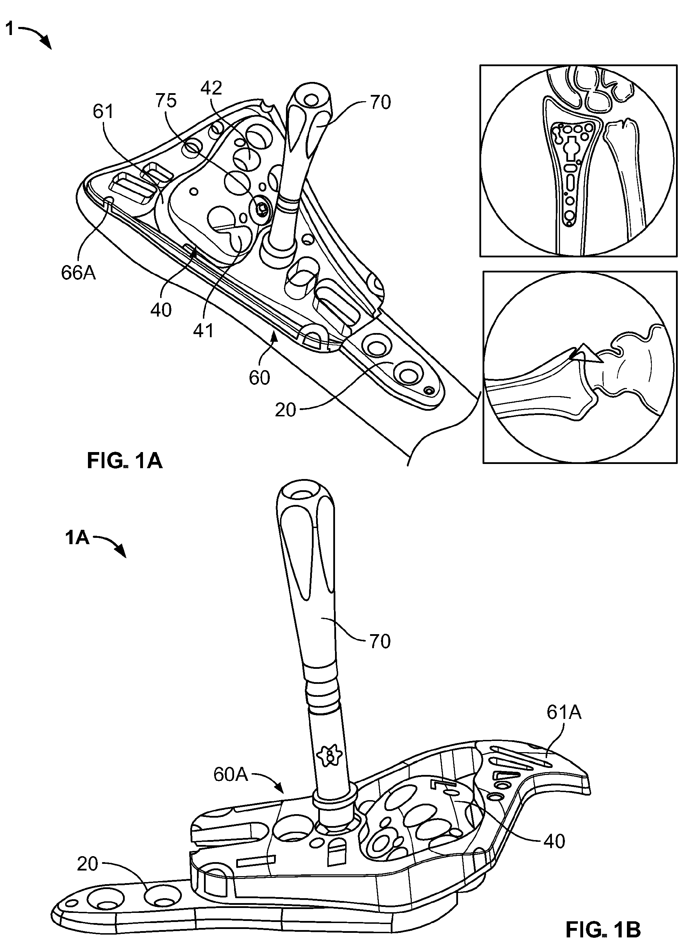

Referring now to the figures, as shown in FIG. 1A, bone plate alignment system 1 generally includes bone plate 20, aiming block 40, alignment template 60, drill guide 70, set screw 75, and various additional fasteners (not shown) for aligning and fixing the bone plate to bone 10. In the assembly shown, alignment template 60 is placed over bone plate 20, aiming block 40 is inserted within central opening 61 of the alignment template and attached to the plate via set screw 75, and drill guide 70 is threadably attached at an end of the drill guide to the plate.

As shown in FIG. 1B, alternative bone plate alignment system 1A is the same as bone plate alignment system 1 with the exception that bone plate alignment system 1A includes alignment template 60A in place of alignment template 60. Alignment template 60A is substantially similar to alignment template 60 with the notable exception that alignment template 60A includes sharper corner portion 61A for covering the radial styloid of a bone under repair.

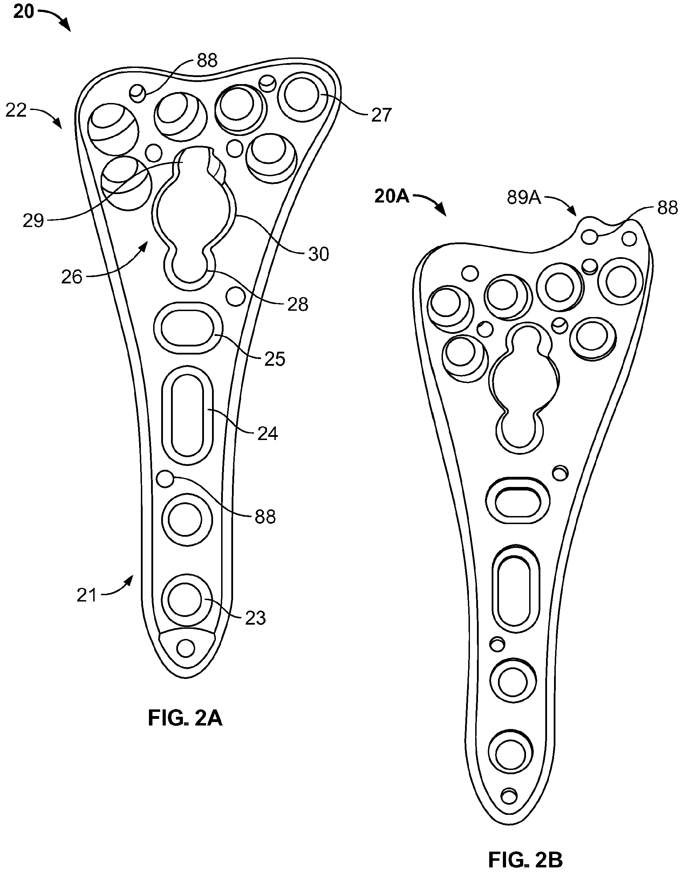

Referring now to FIG. 2A, bone plate 20 includes elongated proximal portion 21 and generally fan-shaped distal portion 22 extending from the proximal portion. Proximal portion 21 includes a plurality of fastener-receiving holes 23, longitudinal slot 24 in the form of an oblong hole distal to the fastener-receiving holes of the proximal portion, and a plurality of small holes 88. Lateral slot 25 is an oblong hole distal to longitudinal slot 24 that extends at a transition from proximal portion 21 to distal portion 22 and in an orthogonal direction to the direction that the longitudinal slot extends. Longitudinal slot 24 and lateral slot 25 have a width that matches a diameter of fastener-receiving holes 23. In this manner, fastener-receiving holes 23, longitudinal slot 24, and lateral slot 25 are configured for receiving the same fasteners in order to attach bone plate 20 to a bone onto which the bone plate is placed. Small holes 88 have the same or approximately the same diameter as the diameter of K-wires that the small holes are configured to receive along respective axes of the small holes.

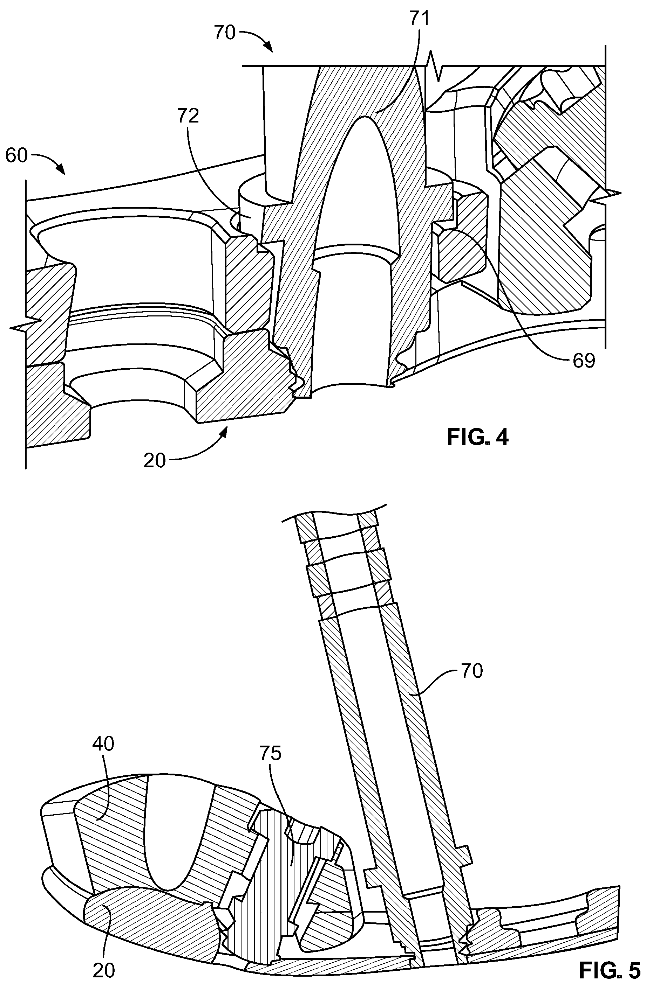

Distal portion 22 includes central window 26 distal to lateral slot 25, a plurality of fastener-receiving holes 27 distal and lateral to the central window, and additional small holes 88. Central window 26 includes proximal partial hole 28, distal partial hole 29 on longitudinally opposed ends of the central window, and central section 30 extending between the proximal and the distal partial holes. Central section 30 includes two opposing arcs that each extend outwardly from and between respective ends of proximal partial hole 28 and distal partial hole 29 such that the central section is wider than the proximal and the distal partial holes. In this manner, central window 26, and in particular central section 30, provides a functional window for bone fracture visualization. In this example, proximal partial hole 28 is threaded for attachment to drill guide 70 (see FIGS. 2A and 4), such as for use during positioning of bone plate 20, and distal partial hole 29 is threaded for receipt of set screw 75 to attach aiming block 40 to the bone plate (see FIG. 1A). Proximal partial hole 28 and distal partial hole 29 are also configured for attachment to respective threaded heads of fasteners for fixation to an underlying bone part via the respective fasteners.

As shown, fastener-receiving holes 27 include holes on opposite sides of distal hole of central window 26 in which the holes and the distal hole of the central window define a first set of holes that lie along a transverse axis transverse to a longitudinal axis defined by proximal portion 21. As further shown, fastener-receiving holes 27 further include a second set of holes that are distal to the first set of holes of distal portion 22 and that lie along a transverse axis transverse to the longitudinal axis defined by proximal portion 21. As in the example shown, all of fastener-receiving holes 23, 27 may be unthreaded holes configured for plastic deformation upon receipt of a threaded head of a fastener into any such holes at an angle selected by the surgeon, such as the holes of the VariAx 2^{®} Variable Angle Locking Plate System with SmartLock technology by Stryker Corporation. In other arrangements, fastener-receiving holes 23, 27 may be threaded or unthreaded as desired. In the example shown, each of the four additional small holes 88 of distal portion 22 are located to be adjacent to respective lateral pairs of each of the first and the second sets of holes of fastener-receiving holes 27.

In an alternative arrangement, as shown in FIG. 2B, bone plate 20A is the substantially the same as bone plate 20 with the notable exceptions that bone plate 20A includes plate extension portion 89A and a modified contour along a distal end of the bone plate. As in this example, plate extension portion 89A may have a low profile and be pre-bent whereby plate extension portion 89A is configured to buttress volar-ulnar corner fragments. As shown, plate extension portion 89A includes two additional small holes 88. These additional small holes are configured to receive additional K-wires that extend into bone 10 or to allow suturing of the volar capsule.

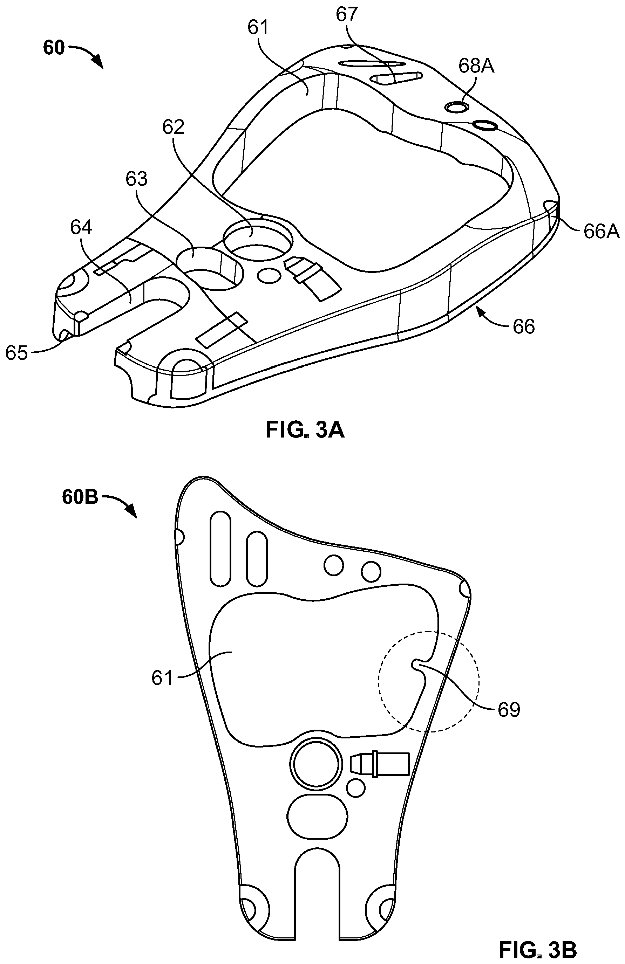

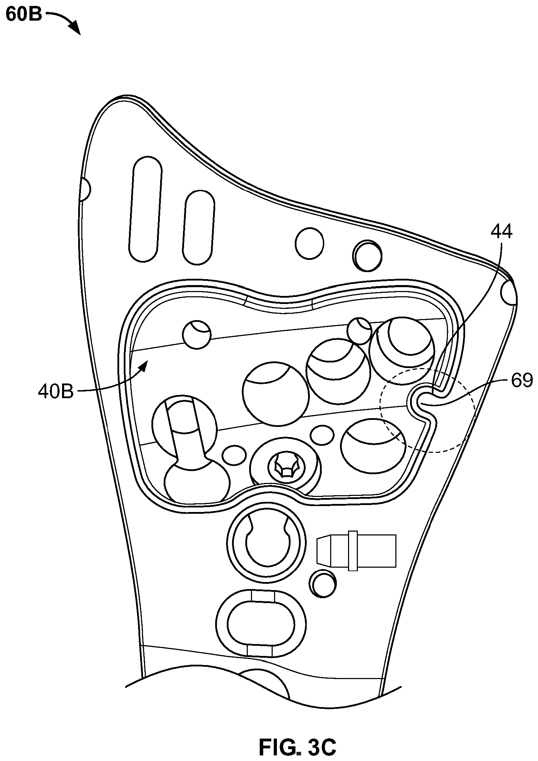

As shown in FIG. 3A, alignment template 60 is generally fan-shaped and, besides central opening 61, includes guide hole 62, guide lateral slot 63 distal to the guide hole, guide open slot 64 distal to the guide lateral slot, and track 65 extending along a bone-facing side of the alignment template. As in this example, except for the proximal end of alignment template 60, track 65 may define a cavity that extends along aiming block 40 such that an outer rim of the bone plate 20 abuts and corresponds to the track when alignment template 60 is placed onto the bone plate. In this manner, bone plate 20 is set in a fixed position within and relative to alignment template 60 and thereby longitudinal and lateral movements of alignment template 60 cause corresponding movements of the bone plate. Central opening 61 has a profile corresponding to a profile of aiming block 40. Guide hole 62 overlies proximal partial hole 28, guide lateral slot 63 overlies lateral slot 25, and guide open slot 64 overlies longitudinal slot 24 when alignment template 60 is placed onto bone plate 20.

A majority of alignment template 60 is radiolucent. In some arrangements, alignment template 60 is transparent or at least translucent. Alignment template 60 includes radiopaque frame 66 that extend on and around the outer rim of the alignment template. Alignment template 60 further includes small guide slots 67 and small guide holes 68 configured for receipt of K-wires that extend through the alignment template and distal to bone plate 20 when alignment template 60 is placed onto the bone plate. Alignment template 60 still further includes small guide hole 68A configured for receipt of a K-wire that extends through the alignment template and that overlies and is aligned with a corresponding small hole 88 of proximal portion 21 of bone plate 20 adjacent to lateral slot 25.

In an alternative arrangement, as shown in FIGS. 3B-3C, alignment template 60B is substantially the same as alignment template 60 with the notable exception that alignment template 60B includes an alignment feature 69. Alignment feature 69 extends into central opening 61 such that an alignment opening 44 of aiming block 40B receives and abuts alignment feature 69 when alignment template 60B is placed onto the bone plate. Alignment feature 69 ensures that a correct sized aiming block 40B is assembled onto bone plate 20 in the proper orientation. Additionally, alignment feature 69 prevents bone plate 20 from being able to move in the volar direction when aiming block 40B is not received in central opening 61 and is not attached to bone plate 20. Although a single alignment feature is shown in the drawings, it is to be understood that multiple features could be included. Moreover, these features could take on many different shapes and sizes to cooperate with similarly sized and shaped openings in the blocks

Referring to FIG. 4, drill guide 70 includes shaft 71 and flange 72 extending outwardly from the shaft. Guide hole 62 of alignment template 60 includes step 69. Flange 72 is located along shaft 71 such that when alignment template 60 is placed onto bone plate 20 and drill guide 70 is attached to the plate, flange 72 of drill guide 70 is spaced apart from step 69 of alignment template 60. In this manner, bone plate 20 is able to move in the dorsal-volar direction and to move relative to alignment template 60.

Referring again to FIG. 1A, aiming block 40 has a first set of guide holes 41, a second set of guide holes 42, and a plurality of small guide holes 43 that each extend through the aiming block. When a middle one of the first set of guide holes 41 of aiming block 40 is attached to bone plate 20 via set screw 75, the first set of guide holes respectively overlie and are aligned with each of the first set of holes 27 of bone plate 20, the second set of guide holes 42 respectively overlie and are aligned with each of the second set of holes 27 of the bone plate, and the plurality of small guide holes 43 respectively overlie and are aligned with each of small holes 88 of the bone plate. In this manner, fasteners can be inserted through the aiming block 40 and into bone plate 20 and the respective bone part or parts underlying the bone plate to secure the bone plate to the respective bone part or parts. In such arrangements, guide holes 41 and 42 may be configured to receive fasteners intended for fixed or for variable angle fixation to bone plate 20.

With reference to FIG. 5, drill guide 70 is able to be attached to bone plate 20 and, via set screw 75, aiming block 40 is able to be attached to the bone plate while alignment template 60 is removed from bone plate 20 and the remainder of bone plate alignment system 1. In this arrangement, drill guide 70 may act as a "joystick" to move the bone plate 20.

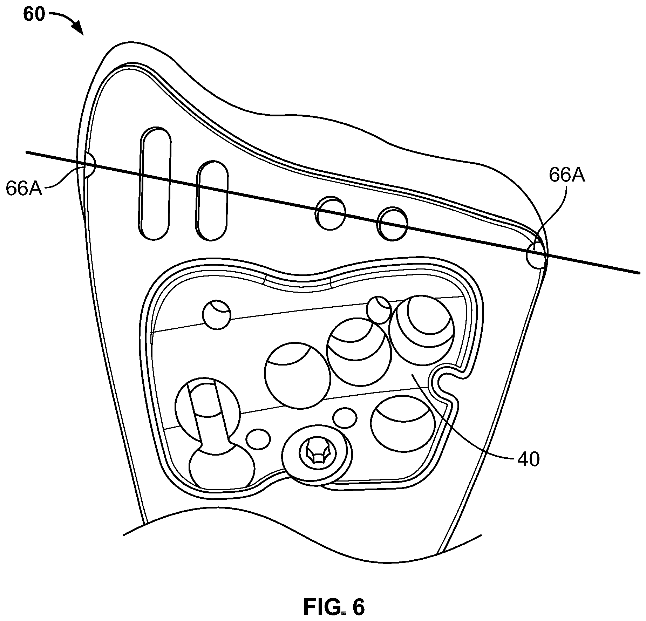

Referring once again to FIG. 1A, to position bone plate 20, the bone plate is placed onto the epiphysis of fractured bone 10. Alignment template 60, placed onto bone plate 20, is positioned such that a distal end of the alignment template visually aligns with the corresponding contour of the distal end of the epiphysis of fractured bone 10. As alignment template 60 is placed onto bone plate 20, the bone plate moves in a corresponding manner to the movement of the alignment template. Alignment of alignment template 60 with bone 10 may be performed without aiming block 40 or with aiming block 40 received in central opening 61 and attached to bone plate 20. To verify the position of alignment template 60 and thereby the position of bone plate 20, anterior-posterior (AP) x-ray and lateral x-rays may be taken (see FIG. 1A). In the AP x-ray (upper right image of FIG. 1A), radiopaque frame 66 outline alignment template 60 to show its relative position to bone 10 such that the surgeon can determine whether the alignment template and thereby bone plate 20 should be moved longitudinally or laterally. In the lateral x-ray (lower right image of FIG. 1A), protruding indicia 66A of radiopaque frame 66 on opposing sides of alignment template 60 (see FIG. 6) should be aligned such that the indicia should appear as a single indicia. If all or part of both protruding indicia 66A appear in the lateral x-ray image, then alignment template 60 and thereby bone plate 20 should be moved appropriately relative to bone 10.

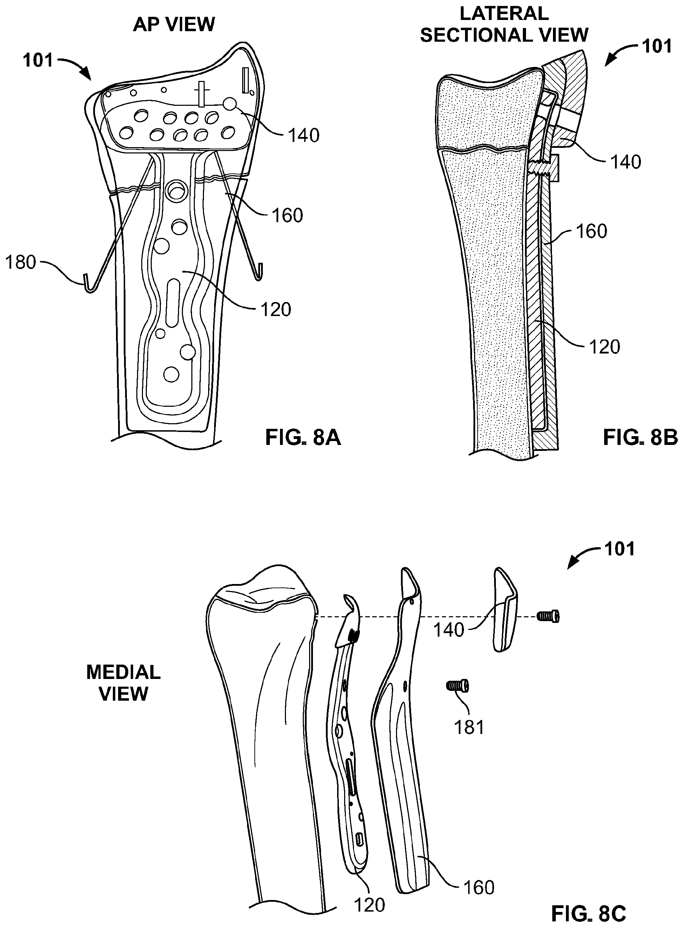

Referring now to FIG. 7 and FIGS. 8A-8C, bone plate alignment system 101 includes bone plate 120, aiming block 140, alignment template 160, K-wires 180, and fixation screws 181. Bone plate alignment system 101 is similar to bone plate alignment system 1 with the notable exception that alignment template 160 excludes a central opening like that of central opening 61 of alignment template 60 such that aiming block 140 covers a distal portion of alignment template 160 when aiming block 140 is attached to bone plate 120 (or other types of bone plates such as bone plate 20), which in this example is a T-shaped distal radius plate. With specific reference to FIG. 8B, alignment template 160 closely surrounds, and in some arrangements tightly surrounds or even has a snap fit with, bone plate 120 such that movement of the alignment template moves bone plate 120 and, if attached to bone plate 120, aiming block 140 as well.

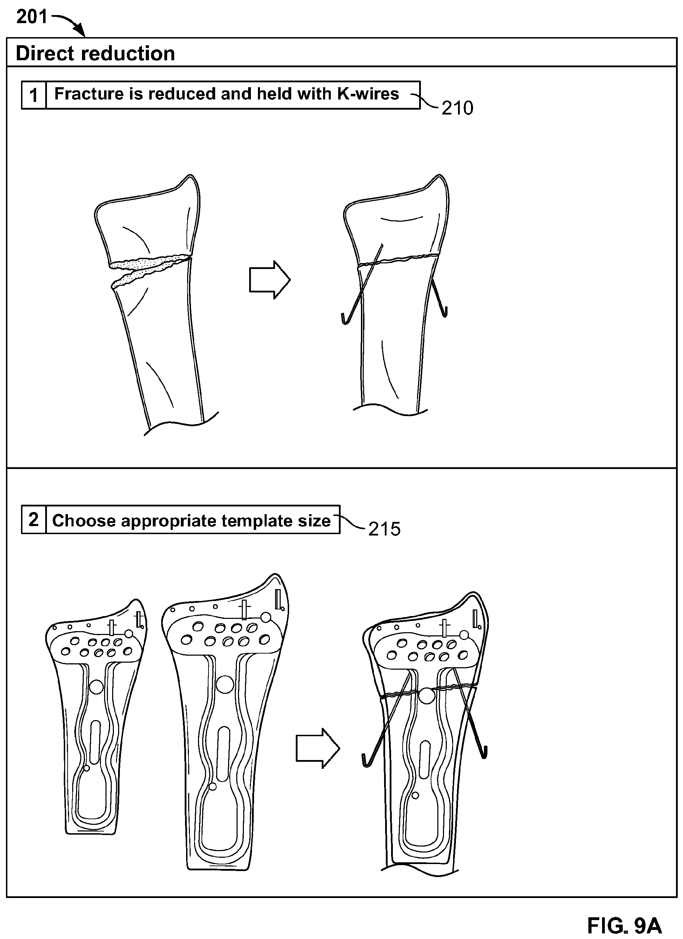

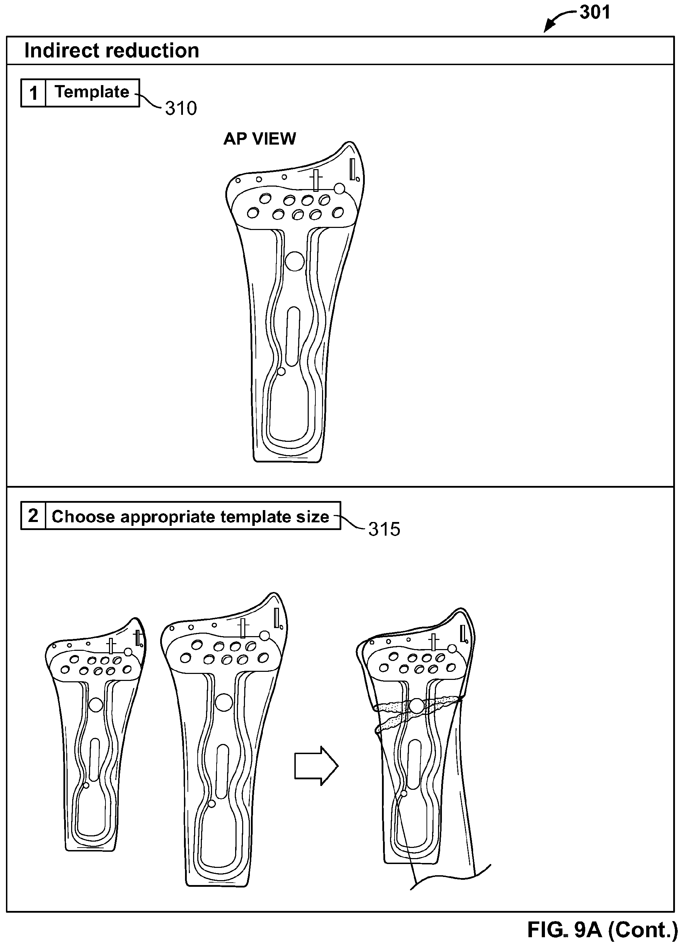

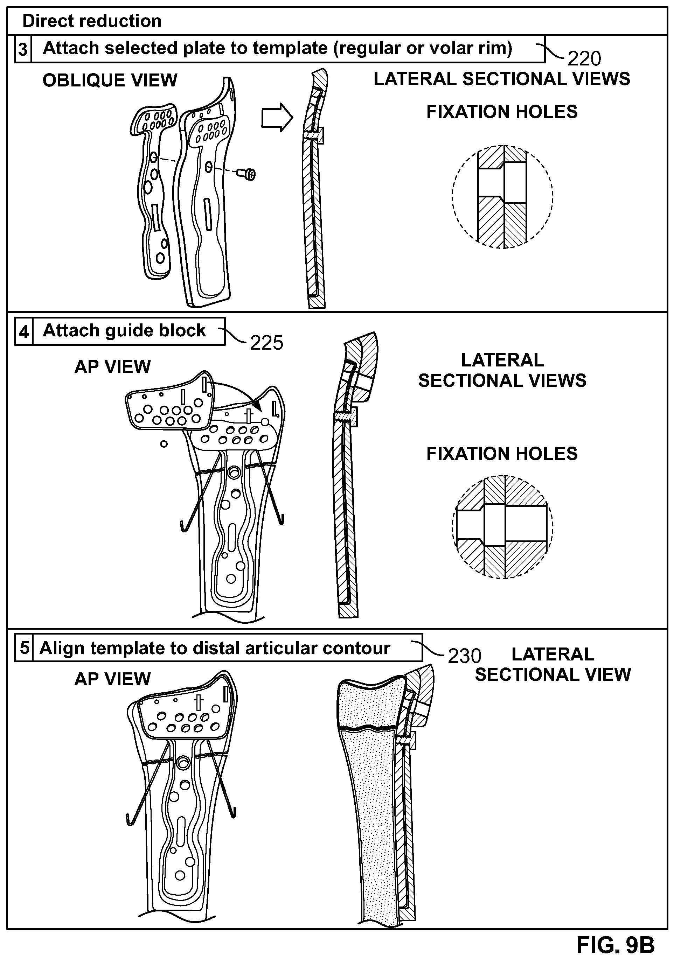

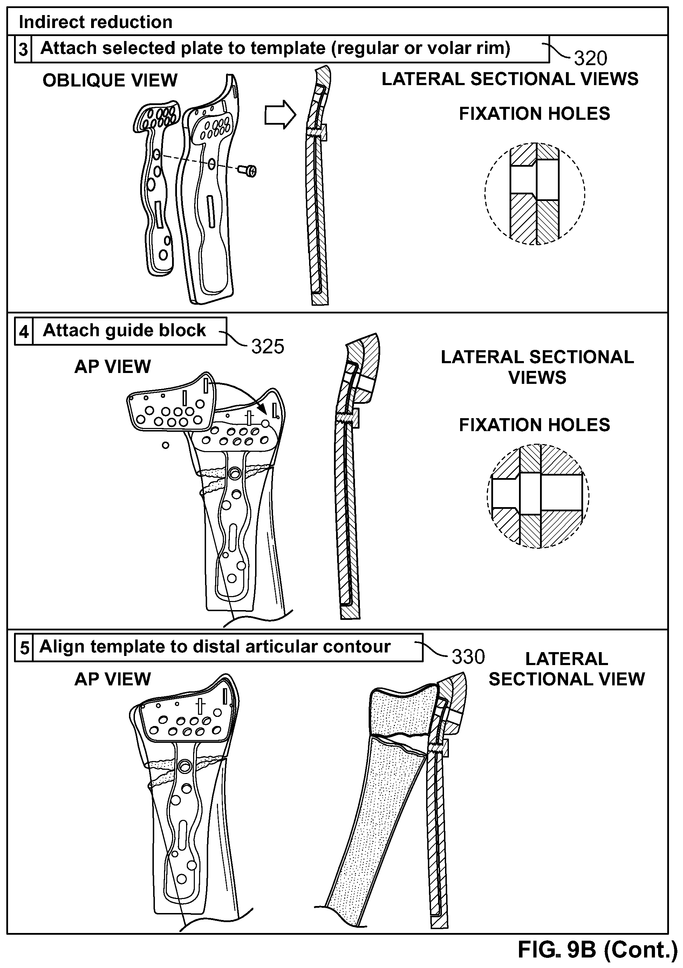

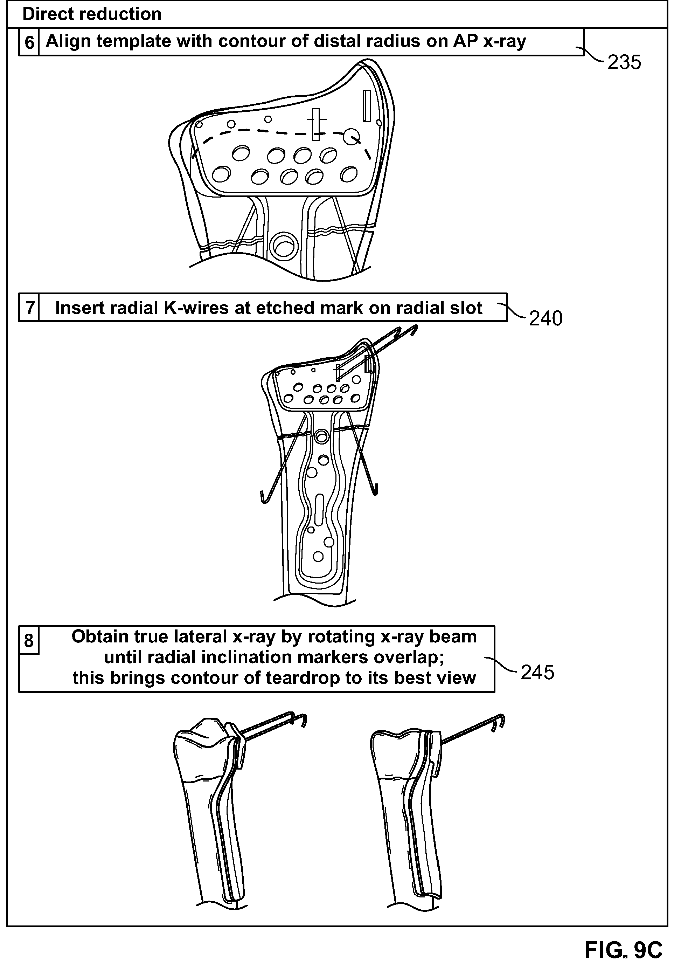

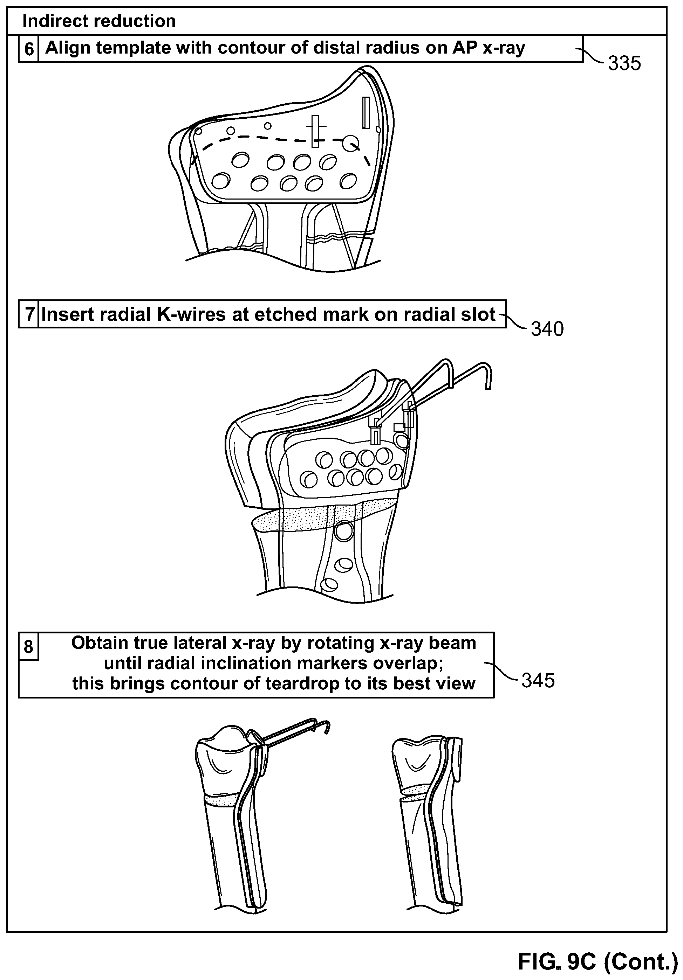

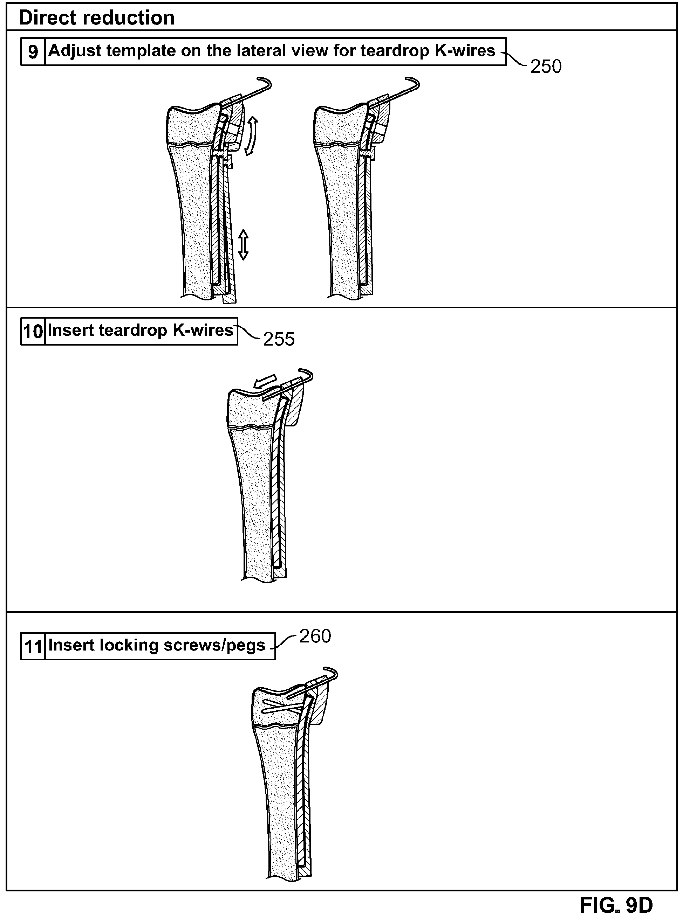

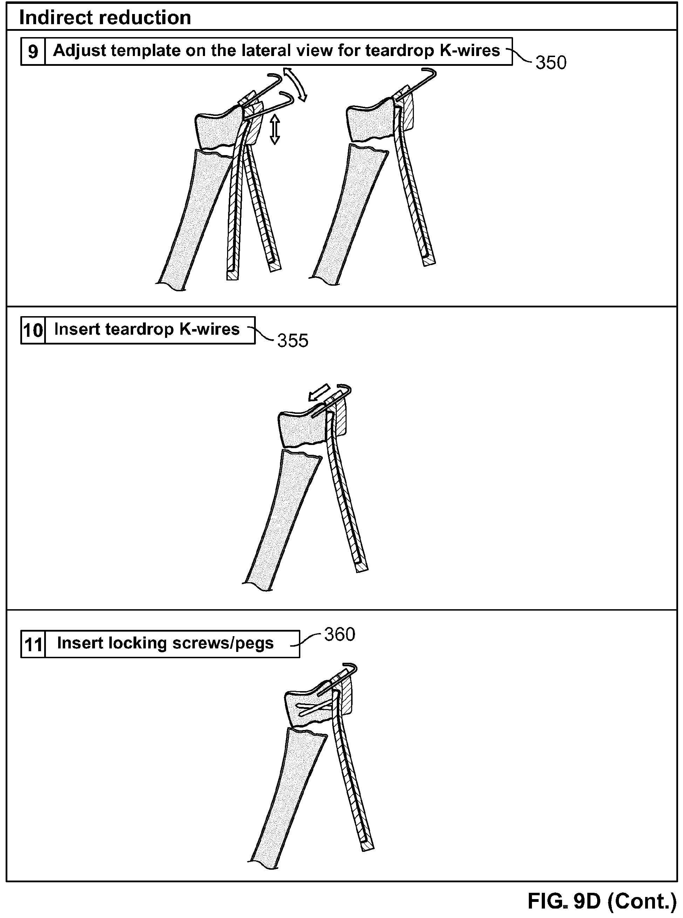

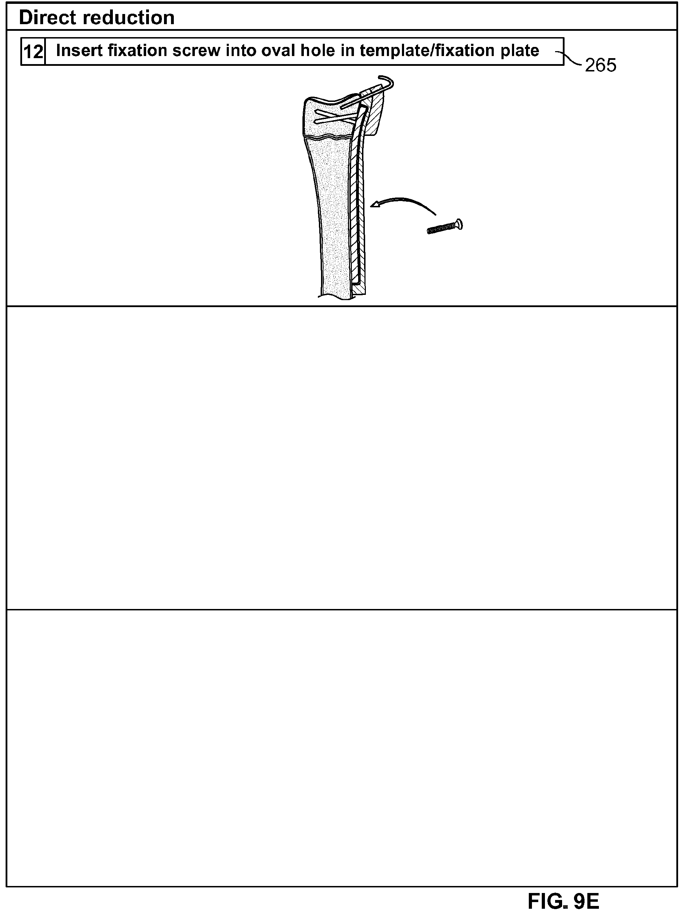

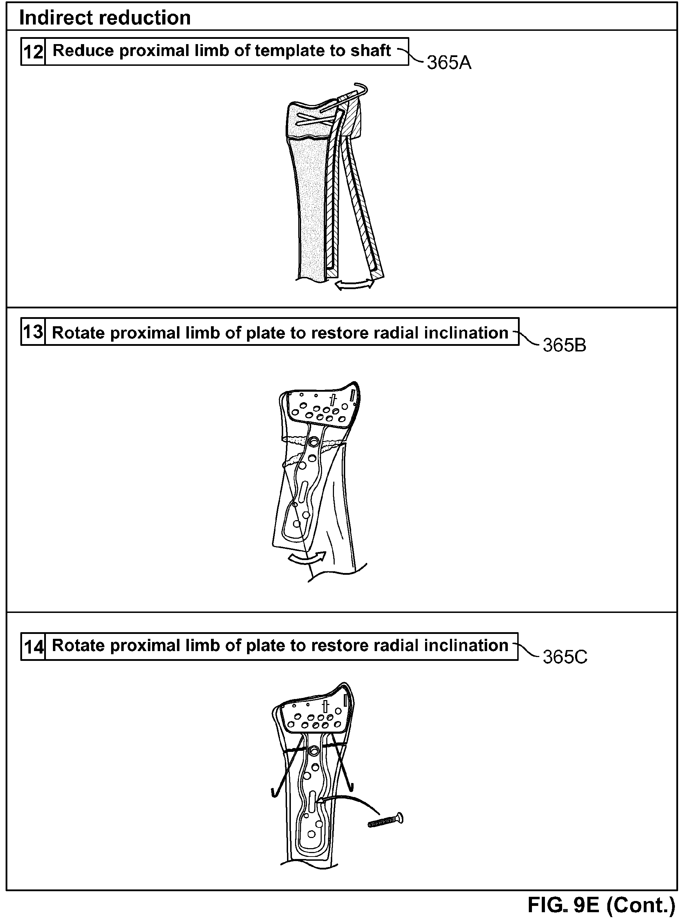

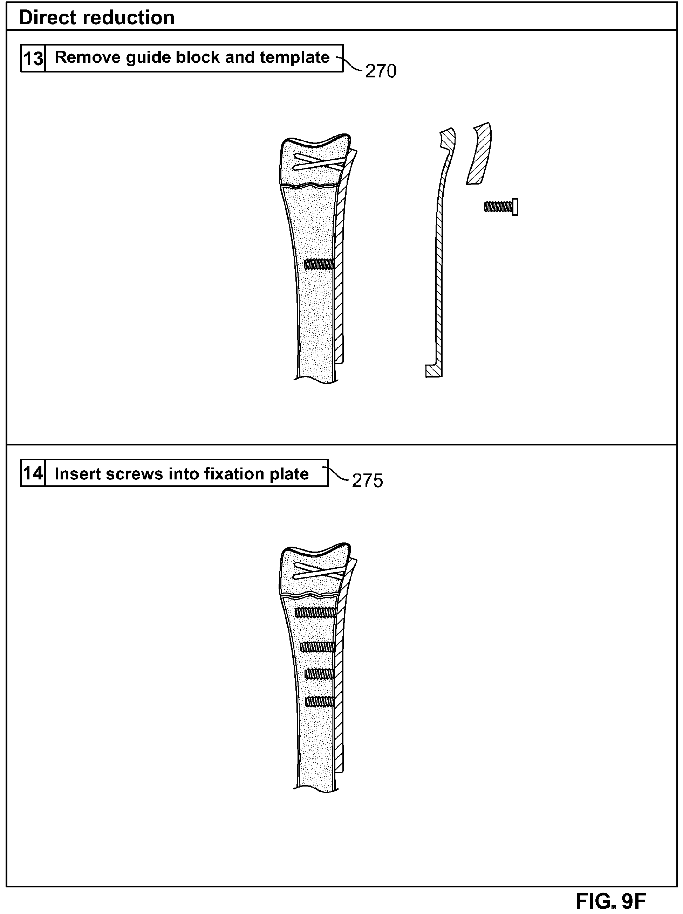

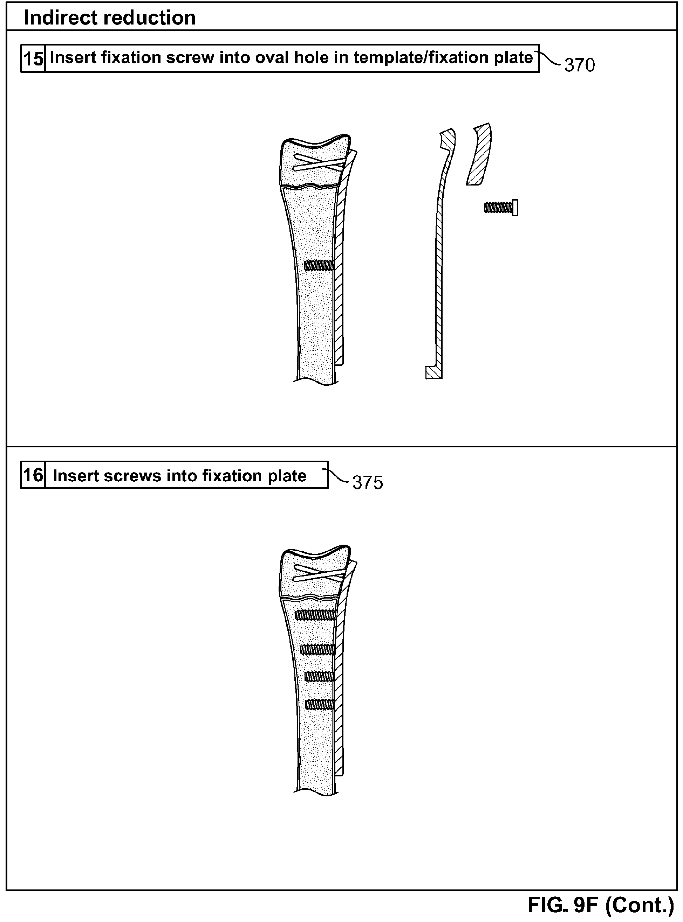

Referring now to FIGS. 9A-9F, in a method of use useful for understanding the invention and not being a part thereof, a bone alignment system as described herein such as bone plate alignment system 101 may be reduce a bone fracture using a bone plate to repair the fracture. In direct reduction process 201, at step 210, a bone fracture is reduced and held with K-wires. At step 215, an appropriate template size of an alignment template, e.g., alignment template 160, is determined based on the bone to be repaired. At step 220, a bone plate corresponding to the selected alignment template, e.g., plate 120, is selected. Such bone plate may be a regular or volar rim plate. At step 225, a guide block, e.g., aiming block 140, is attached to the bone plate. At step 230, the alignment template is moved to a distal articular contour of the bone such that the bone plate moves in a corresponding manner to a corresponding location on the bone. At step 235, the alignment template is aligned with a contour of the bone on an AP x-ray. At step 240, K-wires, e.g., K-wires 180, are inserted at etch marks on the template at the radial styloid. At step 245, a true lateral x-ray image is obtained by rotating the x-ray beam until the radial inclination markers overlap and thereby bring the contour of the teardrop to its optimal view. At step 250, the alignment template is adjusted based on the lateral x-ray image for use with teardrop K-wires. At step 255, teardrop K-wires are inserted into the distal bone part. At step 260, locking screws or pegs are inserted into distal holes of the bone plate and the underlying distal bone part. In this step, as shown, the screws or pegs may be inserted through the aiming block and the alignment template. At step 265, a fixation screw is inserted into an oval hole, e.g., longitudinal hole, in the alignment template. At step 270, the guide block and the alignment template are removed from the bone plate by removing a fastener attaching the alignment template to the guide block. At step 275, additional fixation screws, as desired by the surgeon, are inserted into the bone plate and the underlying bone.

In contrast, in indirect reduction process 301, at steps 310 and 315, an appropriate template size of an alignment template, e.g., alignment template 160, is determined based on the bone to be repaired. At step 320, a bone plate corresponding to the selected alignment template, e.g., plate 120, is selected. Such bone plate may be a regular or volar rim plate. At step 325, a guide block, e.g., aiming block 140, is attached to the bone plate. At step 330, the alignment template is moved to a distal articular contour of the bone such that the bone plate moves in a corresponding manner to a corresponding location on the bone. At step 335, the alignment template is aligned with a contour of the bone on an AP x-ray. At step 340, K-wires, e.g., K-wires 180, are inserted at etch marks on the template at the radial styloid. At step 345, a true lateral x-ray image is obtained by rotating the x-ray beam until the radial inclination markers overlap and thereby bring the contour of the teardrop to its optimal view. At step 350, the alignment template is adjusted based on the lateral x-ray image for use with teardrop K-wires. At step 355, teardrop K-wires are inserted into the distal bone part. At step 360, locking screws or pegs are inserted into distal holes of the bone plate and the underlying distal bone part. At step 365A, a proximal portion of the alignment template, and thereby the bone plate, is rotated towards the shaft of the bone until a proximal portion of the bone plate lies along the shaft of the bone. At steps 365B and 365C, the proximal portion of the alignment template, and thereby the bone plate, is rotated laterally to restore the radial inclination of the bone and place the distal portion of the bone and the shaft of the bone into abutment along the bone fracture. At step 370, the guide block and the alignment template are removed from the bone plate by removing a fastener attaching the alignment template to the guide block. At step 375, additional fixation screws, as desired by the surgeon, are inserted into the bone plate and the underlying bone.

It is to be understood that the disclosure set forth herein includes any possible combinations of the particular features set forth above, whether specifically disclosed herein or not. For example, where a particular feature is disclosed in the context of a particular aspect, arrangement, configuration, or embodiment, that feature can also be used, to the extent possible, in combination with and/or in the context of other particular aspects, arrangements, configurations, and embodiments of the invention, and in the invention generally.

Furthermore, although the invention disclosed herein has been described with reference to particular features, it is to be understood that these features are merely illustrative of the principles and applications of the present invention. It is therefore to be understood that numerous modifications may be made to the illustrative embodiments and that other arrangements may be devised without departing from the scope of the present invention. In this regard, the present invention encompasses numerous additional features in addition to those specific features set forth in the claims below.

## Claims

1. A bone plate alignment template (60; 160) for placement on a bone (10), comprising:
an at least radiolucent main body including a bone-facing lower surface, an upper surface opposite the lower surface, and a cavity (65) extending inwardly from the lower surface towards the upper surface, wherein the cavity (65) is configured to receive a portion of a bone plate (20; 120); and
a radiopaque outer portion extending on and around an outer rim of the main body.

2. The bone plate alignment template (60; 160) of claim 1, further comprising
distal and proximal ends; and
a central opening (61) within the main body, the central opening (61) extending across a majority of a length and a majority of a width of the distal end.

3. The bone plate alignment template (60; 160) of claim 2, wherein the distal end defines a generally fan-shaped portion and includes an extension portion extending distally from one side of the fan-shaped portion.

4. The bone plate alignment template (60; 160) of claim 3, further comprising a distal hole (63) extending through the central portion, wherein the distal hole is between the extension portion and the central opening (61), and wherein the distal hole is an oblong hole.

5. The bone plate alignment template (60; 160) of any one of claims 2-4, further comprising a proximal hole (68A) proximal the central opening (61).

6. The bone plate alignment template (60; 160) of any one of claims 1-5, wherein the template (60; 160) defines a longitudinal axis, further comprising:
radiopaque indicia (66A) on opposing sides of an edge of the main body; and
an alignment hole through the main body, wherein the radiopaque indicia and a center of the alignment hole lie along an axis transverse to the longitudinal axis.

7. The bone plate alignment template (60; 160) of any one of claims 2-6, wherein the template (60; 160) includes an alignment feature (69) extending from the main body into the central opening (61).

8. An orthopedic repair system, comprising:
a bone plate (20; 120); and
the bone plate alignment template (60; 160) according to claim 1,
wherein the cavity (65) includes a wall corresponding to a side surface of the bone plate (20; 120) such that the wall closely surrounds the portion of the bone plate (20; 120) and the bone plate (20; 120) moves either one of or both longitudinally and laterally with the alignment template (60; 160) when the portion of the bone plate (20; 120) is received in the cavity (65).

9. The orthopedic repair system of claim 8, wherein the alignment template (60; 160) includes a central opening (61) and the bone plate (20; 120) includes a set of bone plate holes, further comprising an aiming block (40) configured for receipt in the central opening, wherein the aiming block includes a plurality of block holes configured for alignment with the bone plate holes when the bone plate (20; 120) is received in the cavity (65) of the alignment template (60; 160) and the aiming block is received in the central opening, wherein the aiming block has a profile corresponding to a profile of the central opening, and further comprising a first fastener configured to attach the aiming block to the bone plate (20; 120).

10. The orthopedic repair system of claim 9, wherein the bone plate (20; 120) includes an oblong hole (24, 25) and the alignment template (60; 160) includes a corresponding oblong guide hole (63, 64) proximal to the central opening (61) and configured for alignment with the oblong hole of the bone plate (20; 120).

11. The orthopedic repair system of any one of claims 8 to 10, wherein the bone plate (20; 120) includes a central window (30) defined in part by a partial hole (28), wherein the alignment template (60; 160) includes a guide hole (62) configured for alignment with the partial hole of the central window when the bone plate (20; 120) is received in the cavity (65) of the alignment template (60; 160), and wherein the partial hole includes threading configured for a threaded connection with a fixation screw having a shank and a threaded head extending from the shank.

12. The orthopedic repair system of any one of claims 8-10, the alignment template (60; 160) including a guide hole (62), further comprising a drill guide (70) including a shaft (71) and a flange (72) extending around the shaft, wherein the drill guide is configured for being simultaneously received through the guide hole of the alignment template (60; 160) and attached to the bone plate (20; 120) such that the alignment template (60; 160) is held between the flange of the drill guide and the bone plate (20; 120).

13. The orthopedic repair system of claim 12, wherein the guide hole of the alignment template (60; 160) includes a step (69), and wherein the step lies between the flange (72) and the bone plate (20; 120) when the drill guide (70) is received through the guide hole and attached to the bone plate (20; 120), and wherein the flange extends from a location along the shaft (71) of the drill guide such that the flange is spaced from the step when the drill guide is received through the guide hole and attached to the bone plate (20; 120).

14. The orthopedic repair system of claim 9 or any one of claims 10-13, when depending on claim 9, wherein the alignment template (60; 160) includes an alignment feature (69) extending into the central opening (61).

15. The orthopedic repair system of claim 14, wherein the alignment feature is configured to be received by an alignment opening of the aiming block (40).

## Patentansprüche

1. Knochenplatten-Ausrichtungsschablone (60; 160) zum Anbringen an einem Knochen (10), umfassend:
einen zumindest röntgentransparenten Hauptkörper, der eine dem Knochen zugewandten Unterseite, eine der Unterseite gegenüberliegende Oberseite und einen Hohlraum (65) enthält, der sich von der Unterseite nach innen in Richtung der Oberseite erstreckt, wobei der Hohlraum (65) ausgebildet ist, um einen Abschnitt einer Knochenplatte (20; 120) aufzunehmen; und
einen röntgendichten Außenabschnitt, der sich an und um einen Außenrand des Hauptkörpers herum erstreckt.

2. Knochenplatten-Ausrichtungsschablone (60; 160) nach Anspruch 1, ferner umfassend:
distale und proximale Enden; und
eine zentrale Öffnung (61) innerhalb des Hauptkörpers, wobei sich die zentrale Öffnung (61) über einen Großteil einer Länge und einen Großteil einer Breite des distalen Endes erstreckt.

3. Knochenplatten-Ausrichtungsschablone (60; 160) nach Anspruch 2, wobei das distale Ende einen im Wesentlichen fächerförmigen Abschnitt definiert und einen Verlängerungsabschnitt enthält, der sich distal von einer Seite des fächerförmigen Abschnitts aus erstreckt.

4. Knochenplatten-Ausrichtungsschablone (60; 160) nach Anspruch 3, ferner umfassend ein distales Loch (63), das sich durch den zentralen Abschnitt erstreckt, wobei sich das distale Loch zwischen dem Verlängerungsabschnitt und der zentralen Öffnung (61) befindet und wobei das distale Loch ein längliches Loch ist.

5. Knochenplatten-Ausrichtungsschablone (60; 160) nach einem der Ansprüche 2 bis 4, ferner umfassend ein proximales Loch (68A) proximal zur zentralen Öffnung (61).

6. Knochenplatten-Ausrichtungsschablone (60; 160) nach einem der Ansprüche 1 bis 5, wobei die Schablone (60; 160) eine Längsachse definiert und ferner umfasst:
röntgendichte Markierungen (66A) auf gegenüberliegenden Seiten einer Kante des Hauptkörpers; und
ein Ausrichtungsloch durch den Hauptkörper, wobei die röntgendichten Markierungen und ein Mittelpunkt des Ausrichtungslochs entlang einer Achse quer zu der Längsachse liegen.

7. Knochenplatten-Ausrichtungsschablone (60; 160) nach einem der Ansprüche 2 bis 6, wobei die Schablone (60; 160) ein Ausrichtungsmerkmal (69) enthält, das sich von dem Hauptkörper in die zentrale Öffnung (61) erstreckt.

8. Orthopädisches Reparatursystem, umfassend:
eine Knochenplatte (20; 120); und
die Knochenplatten-Ausrichtungsschablone (60; 160) nach Anspruch 1,
wobei der Hohlraum (65) eine Wand enthält, die einer Seitenfläche der Knochenplatte (20; 120) entspricht, so dass die Wand den Abschnitt der Knochenplatte (20; 120) eng umschließt und sich die Knochenplatte (20; 120) entweder in Längsrichtung oder in Querrichtung oder in beiden Richtungen zusammen mit der Ausrichtungsschablone (60; 160) bewegt, wenn der Abschnitt der Knochenplatte (20; 120) in dem Hohlraum (65) aufgenommen ist.

9. Orthopädisches Reparatursystem nach Anspruch 8, wobei die Ausrichtungsschablone (60; 160) eine zentrale Öffnung (61) enthält und die Knochenplatte (20; 120) einen Satz von Knochenplattenlöchern enthält, ferner umfassend einen Zielblock (40), der zur Aufnahme in der zentralen Öffnung ausgebildet ist, wobei der Zielblock eine Vielzahl von Blocklöchern enthält, die zur Ausrichtung mit den Knochenplattenlöchern ausgebildet sind, wenn die Knochenplatte (20; 120) in den Hohlraum (65) der Ausrichtungsschablone (60; 160) aufgenommen ist und der Zielblock in der zentralen Öffnung aufgenommen ist, wobei der Zielblock ein Profil aufweist, das einem Profil der zentralen Öffnung entspricht, und ferner umfassend eine erste Befestigungsvorrichtung, die ausgebildet ist, um den Zielblock an der Knochenplatte (20; 120) zu befestigen.

10. Orthopädisches Reparatursystem nach Anspruch 9, wobei die Knochenplatte (20; 120) ein längliches Loch (24, 25) enthält und die Ausrichtungsschablone (60; 160) ein entsprechendes längliches Führungsloch (63, 64) enthält, das proximal zur zentralen Öffnung (61) angeordnet und zur Ausrichtung mit dem länglichen Loch der Knochenplatte ausgebildet ist (20; 120).

11. Orthopädisches Reparatursystem nach einem der Ansprüche 8 bis 10, wobei die Knochenplatte (20; 120) ein zentrales Fenster (30) enthält, das teilweise durch ein Teilloch (28) definiert ist, wobei die Ausrichtungsschablone (60; 160) ein Führungsloch (62) enthält, das zur Ausrichtung mit dem Teilloch des zentralen Fensters ausgebildet ist, wenn die Knochenplatte (20; 120) in den Hohlraum (65) der Ausrichtungsschablone (60; 160) aufgenommen ist, und wobei das Teilloch ein Gewinde enthält, das für eine Gewindeverbindung mit einer Fixierungsschraube ausgebildet ist, die einen Schaft und einen sich von dem Schaft erstreckenden Gewindekopf aufweist.

12. Orthopädisches Reparatursystem nach einem der Ansprüche 8 bis 10, wobei die Ausrichtungsschablone (60; 160) ein Führungsloch (62) enthält und ferner eine Bohrführung (70) umfasst, die einen Schaft (71) und einen sich um den Schaft herum erstreckenden Flansch (72) enthält, wobei die Bohrführung ausgebildet ist, um gleichzeitig durch das Führungsloch der Ausrichtungsschablone (60; 160) aufgenommen und an der Knochenplatte (20; 120) befestigt zu werden, so dass die Ausrichtungsschablone (60; 160) zwischen dem Flansch der Bohrführung und der Knochenplatte (20; 120) gehalten wird.

13. Orthopädisches Reparatursystem nach Anspruch 12, wobei das Führungsloch der Ausrichtungsschablone (60; 160) eine Stufe (69) enthält und wobei die Stufe zwischen dem Flansch (72) und der Knochenplatte (20; 120) liegt, wenn die Bohrführung (70) durch das Führungsloch aufgenommen und an der Knochenplatte (20; 120) befestigt ist, und wobei sich der Flansch von einer Stelle entlang des Schafts (71) der Bohrführung aus derart erstreckt, dass der Flansch von der Stufe beabstandet ist, wenn die Bohrführung durch das Führungsloch aufgenommen und an der Knochenplatte (20; 120) befestigt ist.

14. Orthopädisches Reparatursystem nach Anspruch 9 oder einem der Ansprüche 10 bis 13, wenn diese von Anspruch 9 abhängen, wobei die Ausrichtungsschablone (60; 160) ein Ausrichtungsmerkmal (69) enthält, das sich in die zentrale Öffnung (61) erstreckt.

15. Orthopädisches Reparatursystem nach Anspruch 14, wobei das Ausrichtungsmerkmal ausgebildet ist, um von einer Ausrichtungsöffnung des Zielblocks (40) aufgenommen zu werden.

## Revendications

1. Gabarit d'alignement pour plaque vissée (60; 160) destiné à être mis en place sur un os (10), comprenant:
un corps principal au moins perméable aux rayons X comprenant une surface inférieure tournée vers l'os, une surface supérieure en regard de la surface inférieure, et une cavité (65) s'étendant vers l'intérieur depuis la surface inférieure en direction de la surface supérieure, la cavité (65) étant conçue pour recevoir une partie d'une plaque vissée (20; 120); et
une partie externe radio-opaque s'étendant sur et autour d'un rebord externe du corps principal.

2. Gabarit d'alignement pour plaque vissée (60; 160) selon la revendication 1, comprenant en outre:
des extrémités distale et proximale; et
une ouverture centrale (61) à l'intérieur du corps principal, l'ouverture centrale (61) s'étendant sur l'ensemble de la longueur et de la largeur de l'extrémité distale.

3. Gabarit d'alignement pour plaque vissée (60; 160) selon la revendication 2, dans lequel l'extrémité distale délimite une partie présentant globalement la forme d'éventail et comporte une partie en saillie s'étendant de manière distale à partir d'un côté de la partie en forme d'éventail.

4. Gabarit d'alignement pour plaque vissée (60; 160) selon la revendication 3, comprenant en outre un trou distal (63) traversant la partie centrale, le trou distal étant situé entre la partie d'extension et l'ouverture centrale (61), et le trou distal étant un trou oblong.

5. Gabarit d'alignement pour plaque vissée (60; 160) selon l'une quelconque des revendications 2 à 4, comprenant en outre un trou proximal (68A) situé à proximité de l'ouverture centrale (61).

6. Gabarit d'alignement pour plaque vissée (60; 160) selon l'une quelconque des revendications 1 à 5, dans lequel le gabarit (60; 160) définit un axe longitudinal, comprenant en outre:
des repères radio-opaques (66A) situés de chaque côté d'un bord du corps principal; et
un trou d'alignement traversant le corps principal, dans lequel les repères radio-opaques et le centre du trou d'alignement sont situés sur un axe transversal à l'axe longitudinal.

7. Gabarit d'alignement pour plaque vissée (60; 160) selon l'une quelconque des revendications 2 à 6, dans lequel le gabarit (60 ; 160) comporte un élément d'alignement (69) s'étendant depuis le corps principal jusqu'à l'ouverture centrale (61).

8. Système de réparation orthopédique, comprenant:
une plaque vissée (20; 120); et
le gabarit d'alignement pour plaque vissée (60; 160) selon la revendication 1,
dans lequel la cavité (65) comprend une paroi correspondant à une surface latérale de la plaque vissée (20 ; 120), de telle sorte que la paroi entoure étroitement la partie de la plaque vissée (20; 120) et que la plaque vissée (20; 120) se déplace longitudinalement et/ou latéralement, avec le gabarit d'alignement (60; 160) lorsque la partie de la plaque vissée (20 ; 120) est logée dans la cavité (65).

9. Système de réparation orthopédique selon la revendication 8, dans lequel le gabarit d'alignement (60; 160) comprend une ouverture centrale (61) et la plaque vissée (20; 120) comprend un ensemble de trous de plaque vissée, comprenant en outre un bloc de visée (40) conçu pour être logé dans l'ouverture centrale, le bloc de visée comprenant une pluralité de trous de bloc conçus pour s'aligner avec les trous de la plaque vissée lorsque la plaque vissée (20; 120) est logée dans la cavité (65) du gabarit d'alignement (60; 160) et que le bloc de visée est logé dans l'ouverture centrale, le bloc de visée présentant un profil correspondant à celui de l'ouverture centrale, et comprenant en outre une première fixation conçue pour fixer le bloc de visée à la plaque vissée (20; 120).

10. Système de réparation orthopédique selon la revendication 9, dans lequel la plaque vissée (20; 120) comprend un trou oblong (24, 25) et le gabarit d'alignement (60; 160) comprend un trou de guidage oblong correspondant (63, 64) situé à proximité de l'ouverture centrale (61) et conçu pour s'aligner avec le trou oblong de la plaque vissée (20; 120).

11. Système de réparation orthopédique selon l'une quelconque des revendications 8 à 10, dans lequel la plaque vissée (20 ; 120) comprend une fenêtre centrale (30) délimitée en partie par un trou partiel (28), dans lequel le gabarit d'alignement (60 ; 160) comprend un trou de guidage (62) conçu pour s'aligner avec le trou partiel de la fenêtre centrale lorsque la plaque vissée (20 ; 120) est logée dans la cavité (65) du gabarit d'alignement (60 ; 160), et dans lequel le trou partiel comporte un filetage configuré pour une liaison filetée avec une vis de fixation comportant une tige et une tête filetée s'étendant à partir de la tige.

12. Système de réparation orthopédique selon l'une quelconque des revendications 8 à 10, le gabarit d'alignement (60 ; 160) comprenant un trou de guidage (62), comprenant en outre un guide de perçage (70) comprenant une tige (71) et un bord (72) s'étendant autour de la tige, dans lequel le guide de perçage est conçu pour être logé simultanément dans le trou de guidage du gabarit d'alignement (60 ; 160) et être fixé à la plaque vissée (20 ; 120) de telle sorte que le gabarit d'alignement (60 ; 160) soit maintenu entre le rebord du guide de perçage et la plaque vissée (20 ; 120).

13. Système de réparation orthopédique selon la revendication 12, dans lequel le trou de guidage du gabarit d'alignement (60 ; 160) comporte un épaulement (69), et dans lequel l'épaulement se trouve entre le rebord (72) et la plaque vissée (20 ; 120) lorsque le guide de perçage (70) est logé dans le trou de guidage et fixé à la plaque vissée (20 ; 120), et dans lequel le bord s'étend à partir d'un emplacement situé le long de la tige (71) du guide de perçage de telle sorte que le bord soit espacé de l'épaulement lorsque le guide de perçage est inséré à travers le trou de guidage et fixé à la plaque vissée (20 ; 120).

14. Système de réparation orthopédique selon la revendication 9 ou l'une quelconque des revendications 10 à 13, lorsque celle-ci dépend de la revendication 9, dans lequel le gabarit d'alignement (60 ; 160) comprend un élément d'alignement (69) s'étendant dans l'ouverture centrale (61).

15. Système de réparation orthopédique selon la revendication 14, dans lequel l'élément d'alignement est conçu pour être logé dans une ouverture d'alignement du bloc de visée (40).
